# Europäisches Patentamt
# European Patent Office
# Office européen des brevets

(11) Veröffentlichungsnummer : **0 326 026 B1**

(12) **EUROPÄISCHE PATENTSCHRIFT**

(45) Veröffentlichungstag der Patentschrift :
**17.06.92 Patentblatt 92/25**

(51) Int. Cl.$^5$ : **A61K 9/14,** A61K 9/16, A61K 9/50, A61K 47/02, A61K 47/26

(21) Anmeldenummer : **89100908.6**

(22) Anmeldetag : **20.01.89**

(54) **Stabiles Gemisch mit einem Gehalt oxidationsempfindlicher Verbindungen, Verfahren zu seiner Herstellung und Verwendung einerKombination von Stoffen zur Stabilisierung oxidationsempfindlicher Verbindungen.**

(30) Priorität : **29.01.88 JP 17232/88**

(43) Veröffentlichungstag der Anmeldung :
**02.08.89 Patentblatt 89/31**

(45) Bekanntmachung des Hinweises auf die Patenterteilung :
**17.06.92 Patentblatt 92/25**

(84) Benannte Vertragsstaaten :
**BE CH DE FR GB IT LI NL**

(56) Entgegenhaltungen :
EP-A- 0 074 050
STN INTERNATIONAL INFORMATION SERVICES DATENBANK: CHEMICAL ABSTRACTS, Band 101, Nr. 18, Zusammenfassung Nr. 157695g,Columbus, Ohio, US; & JP-A-59 122 424 (RIKEN VITAMIN CO., LTD) 14-07-1984
STN INTERNATIONAL INFORMATION SERVICES DATENBANK: CHEMICAL ABSTRACTS, Band 107, Nr. 15, Zusammenfassung Nr. 133022h,Columbus, Ohio, US; & JP-A-62 111 676 (NIPPON OILS AND FATS CO., LTD) 22-05-1987

(56) Entgegenhaltungen :
STN INTERNATIONAL INFORMATION SERVICES DATENBANK: CHEMICAL ABSTRACTS, Band 102, Nr. 13, Zusammenfassung Nr. 111878m,Columbus, Ohio, US; & JP-A-59 204 696 (MITSUI PHARMACEUTICALS, INC.; OMICHI, TERANOBU) 20-11-1984
STN INTERNATIONAL INFORMATION SERVICES DATENBANK: CHEMICAL ABSTRACTS, Band 82, Nr. 24, Zusammenfassung Nr. 160258m, Columbus,Ohio, US; & JP-A-50 010 289 (FUJISAWA PHARMACEUTICAL CO., LTD) 01-02-1975

(73) Patentinhaber : **BASF Aktiengesellschaft Carl-Bosch-Strasse 38 W-6700 Ludwigshafen (DE)**

(72) Erfinder : **Fukamachi, Chiharu 108 Ohazaueda Uedashi Naganoken (JP)**
Erfinder : **Schumacher, Horst, Dr. Battenberger Weg 4 W-6719 Bobenheim am Berg (DE)**
Erfinder : **Bewert, Wolfgang, Dr. Lorscher Ring 8 c W-6710 Frankenthal (DE)**
Erfinder : **Schneider, Joachim, Dr. Plauserstrasse 17 W-6719 Weisenheim am Berg (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein stabiles Gemisch mit einem Gehalt oxidationsempfindlicher Verbindungen, ein Verfahren zur Herstellung des Gemischs, sowie die Verwendung einer Kombination von Stoffen zur Stabilisierung oxidationsempfindlicher Verbindungen.

Wenn oxidationsempfindliche Verbindungen, insbesondere fettlösliche Vitamine, mit Luft in Kontakt kommen, erfolgt eine ungewollte Oxidation. Hierdurch nimmt die Qualität der diese empfindlichen Verbindungen enthaltenden Produkte ab. Insbesondere wenn oxidationsempfindliche Verbindungen in Nahrungs- und Futtermitteln oder im Pharmabereich verwendet werden, kommt es während der Lagerung zur Zerstörung der oxidationsempfindlichen Verbindungen durch Oxidation, und der im Produkt verbleibende Anteil an diesen Verbindungen sinkt.

Um diese Oxidation zu verhindern, kann man beispielsweise Vitamine, Öle und Fette mit einer Kombination von Antioxidatien und Sequestrierungsmitteln behandeln, vgl. E. Furia, "Handbook of Food Additives", 2. Edition, Seiten 271 und 274 bis 281 (1975). In dieser Literaturstelle wird berichtet, daß die kombinierte Wirkung von Sequestrierungsmitteln und Antioxidantien synergistisch sei. Als Sequestrierungsmittel werden eine Vielzahl unterschiedlicher Verbindungen vorgeschlagen, so beispielsweise Salze der Essigsäure, Salze der Zitronensäure, Ethylendiamintetraacetat, Calciumphytat, Natriumthiosulfat und dgl. In der Literaturstelle sind auch Triglyceride erwähnt, allerdings nicht als Mittel zur Stabilisierung, sondern vielmehr als zu stabilisierende Substrate, vgl. ibid Seite 280.

In der JP-A-59204-696 wird eine Kombination von Phytinsäure, dl-$\alpha$-Toccopherol, natürliches Vitamin E oder Vitamin C, als Antioxidans für Öle, Fettsäuren und Nahrungsmittel, die diese Bestandteile enthalten, vorgeschlagen.

Zur Stabilisierung von Vitamin A werden verschiedene Arbeitsweisen vorgeschlagen. So kann man Vitamin A in den Essigsäureester oder einen Fettsäureester, z.B. Palmitinsäureester, überführen und gibt einen synthetischen Oxidationsinhibitor zu. Als Oxidationsinhibitoren kommen hierbei Dibutylhydroxytoluol (BHT), Butylhydroxyanisol (BHA), Nordihydroguajaretsäure (NDGA), Propylgalat (PG) oder N,N′-Diphenylparaphenylendiamin (DPPD) in Betracht. Bei einer anderen Arbeitsweise zur Stabilisierung von Vitamin A überzieht man Vitamin A mit Gelatine, Kasein, Dextrin oder anderen Stoffen, um den Kontakt mit der Luft zu unterbrechen und stellt dann Beadlets oder Pulver her. Nach einer weiteren Arbeitsweise kann man Vitamin A in Gegenwart natürlicher phenolischer Substanzen und Ascorbinsäure in körnige oder pulverförmige Feststoffe überführen und auf diese Weise stabilisieren, vgl. JP-A 122 424-84.

Die Vorschläge des Standes der Technik zur Stabilisierung oxidationsempfindlicher Verbindungen sind jedoch nicht ausreichend, da man weder eine ausreichende Umhüllung der empfindlichen Verbindungen erzielt, noch einen auseichenden Schutz vor den die Oxidation beschleunigenden Schwermetallspuren erreichen kann.

Der Erfindung liegt die Aufgabe zugrunde, ein stabiles Gemisch oxidationsempfindlicher Verbindungen zu schaffen, das stabil und leicht zu handhaben ist und sich insbesondere für Lebensmittel- und Futtermittel sowie für Pharmaanwendungen eignet.

Diese Aufgabe wird gelöst durch Bereitstellung eines stabilen Gemischs mit einem Gehalt oxidationsempfindlicher Verbindungen, enthaltend die oxidationsempfindlichen Verbindungen, Triglyceride, Komplexbildner sowie Umhüllungsstoffe.

Die Kombination von Triglyceriden, Komplexbildnern und Umhüllungsstoffen ergibt überraschend einen vollen Schutz für die oxidationsempfindlichen Verbindungen. Während die Einzelkomponenten der Kombination dieser drei Wirkstoffe nur eine geringe Schutzwirkung aufweisen, erhält man mit der Kombination eine überadditive Schutzwirkung.

Nach einer bevorzugten Ausführungsform der Erfindung enthält das Gemisch zusätzlich übliche Antioxidantien. Hierbei kann es sich beispielsweise um BHT, BHA, Ethoxyquin handeln.

Nach einer weiteren bevorzugten Ausführungsform handelt es sich bei dem Triglycerid um ein Pflanzenöl. Zu den in der Erfindung brauchbaren Triglyceriden gehören Naturprodukte, aus Naturprodukten regenerierte Triglyceride und synthetische Triglyceride. Man kann eines oder mehrere dieser Triglyceride verwenden. Bevorzugt verwendet man Pflanzenöle wie Olivenöl, Sojaöl, Maisöl, Baumwollsaatöl, Sonnenblumenöl, Erdnußöl, Palmöl, Kokosnußöl und Weizenkeimöl, aber man kann auch verschiedene tierische Fette (z.B. Schweinefett, Rinderfett, Schafsfett und Fischöle) und halbsynthetische Fette verwenden, die man durch Umesterung und Recombination, einschließlich Fraktionierung und Hydrierung gewinnt.

Nach einer weiteren bevorzugten Ausführungsform handelt es sich bei dem Komplexbildner um Phytinsäure, Phosphorsäure, Meta-, Pyro- oder Polyphosphorsäure oder um deren Salze. Als Salze kommen insbesondere die Metallsalze (Natrium, Kalium, Calcium) in Betracht.

Zu den Komplexbildnern gehören auch Sequestrierungsmittel für die Schwermetallionen und Chelatbildner. Besonders bei Lebensmitteln kann man die in der Literatur zitierten verwenden [z.B. Thomas E. Furia, "Handbook of Food Aditives", 2. Band, CRC Press, S. 271 bis 281, (1975)]. So kann man beispielsweise auch Carbonsäuren, Polycarbonsäuren, Hydroxycarbonsäuren, Aminosäuren, Weinsäure, Gluconsäure, Zitronensäure und Ethylendiamintetraessigsäure und deren Metallsalze (z.B. Calciumsalz der Phytinsäure, Natriumpyrophosphat, Natriumhexametaphosphat, Natriumtripolyphosphat, Natriumtartrat, Calciumgluconat, Natriumcitrat und Natriumsalz der Ethylendiamintetraessigsäure), Zitronensäureester, Nitrilotriessigsäure-Natriumsalz (NTA-Na), Cystein, Fumarsäure, Maleinsäure und Milchsäure einsetzen.

Man kann auch Kombinationen von Komplexbildnern verwanden.

Nach einer weiteren bevorzugten Ausführungsform der Erfindung handelt es sich bei dem Umhüllungsstoff um einen Filmbildner, insbesondere um Proteine, wie Gelatine, Casein, um Zucker oder Polysaccharide, insbesondere Gummi arabicum, Alginate oder Stärkederivate. Bei den Zuckern kann man insbesondere Saccharose, Glucose und Lactose nennen. Man kann auch Antioxidantien einsetzen, beispielsweise BHT, BHA, Ethoxyquin, sowie Fließ-Hilfsmittel, beispielsweise Siliciumdioxid, Calciumstearat, Calciumphosphat, sowie Füllstoffe, beispielsweise Stärke, Kaolin und Silikate.

Das Verhältnis von Triglyceriden und Komplexbildnern zu den oxidationsempfindlichen Verbindungen, die in den erfindungsgemäßen Gemischen enthalten sein sollen, kann durch die konkret im jeweiligen Beispiel verwendeten Verbindungen variieren. Bezogen auf 1 Gew.-Teil oxidationsempfindliche Verbindungen kann man beispielsweise Triglyceride in einem Bereich von 1 bis 0,01 Gew.-Teilen und Komplexbildner in einem Bereich von 1 bis 0,01 Gew.-Teilen verwenden.

Die so mit Triglyceriden und Komplexbildnern gemischten oxidationsempfindlichen Verbindungen überzieht man mit den Umhüllungsstoffen in der Menge, die zur Umhüllung (englisch: coating) dieser Mischung erforderlich ist. Gegenstand der Erfindung ist auch ein Verfahren zur Herstellung des erfindungsgemäßen Gemischs, das dadurch gekennzeichnet ist, daß man eine Dispersion aus oxidationsempfindlicher Verbindung, Triglycerid, Komplexbildner und Umhüllungsstoff herstellt und diese Dispersion in einen Feststoff überführt.

Im allgemeinen erhält man das erfindungsgemäße Gemisch dadurch, daß man eine wäßrige Dispersion herstellt, indem man als aktive Komponente oxidationsempfindliche Verbindung in Triglyceriden, Komplexbildnern, Umhüllungsstoffen und gegebenenfalls Hilfsmitteln dispergiert, diese Dispersion auf übliche Weise in einen Feststoff überführt und gegebenenfalls einen Trocknungsprozeß anfügt. Nach so einem Verfahren erhält man ein stabiles Gemisch oxidationsempfindlicher Verbindungen, das aus den oxidationsempfindlichen Verbindungen, Umhüllungsstoffen, Triglyceriden und Komplexbildnern besteht.

Bei dem erfindungsgemäßen Verfahren stellt man normalerweise eine wäßrige Dispersion oder wäßrige Emulsion dadurch her, daß man zuerst die wasserlöslichen Substanzen, wie Umhüllungsstoffe, die hydrophile Komponenten sind, und andere Hilfsmittel in Wasser löst, wobei man eine wäßrige Lösung erhält und gleichzeitig die fettlöslischen Substanzen wie die oxidationsempfindlichen Verbindungen und Triglyceride vormischt. Durch Mischen der Lösung mit der Vormischung mit einem geeigneten Rührer oder Homogenisator wird das Ganze dispergiert oder emulgiert. Die erhaltene Dispersion führt man durch Gefriertrocknung, Vakuumtrocknung, Sprühtrocknung oder Konvektionstrocknung in einem körnigen oder pulverförmigen Feststoff über oder granuliert ihn mit einem geeigneten Verfahren und trocknet dann die Körner nochmals.

Bei dem erfindungsgemäßen Verfahren ist anschließend an den Trocknungsprozeß eine zusätzliche Behandlung zur Vernetzung des Überzuges möglich. Im Falle von Gelatine als Umhüllungsstoff kann man beispielsweise mit Aldehyden vernetzen oder eine thermische Vernetzungsbehandlung durchführen. Es ist auch möglich, im Anschluß an den Trocknungsprozeß einen weiteren Überzug aufzubringen, beispielsweise mit Fetten, Paraffin, Wachsen, synthetischen Polymeren, wie Celluloseacetat, Phthalat und dergleichen.

Besonders bevorzugt liegt der Feststoff in Form eines Granulats oder Pulvers vor, wobei eine mittlere Teilchengröße von 100 bis 600 µm besonders günstig ist.

Zu den in der vorliegenden Erfindung genannten oxidationsempfindlichen Verbindungen gehören fettlösliche Vitamine, Carotinoide, Vitamin-A-säure und davon abgeleitete Verbindungen, analoge Retinoide, Aromastoffe (z.B. Zitronenöl), Riechstoffe und dergleichen. Man kann eine oder mehrere dieser Verbindungen verwenden. Zu den fettlöslichen Vitaminen gehören beispielsweise Vitamin A, Vitamin E, Vitamin D, die Vitamine der K-Reihe sowie Gemische dieser Vitamine, die sowohl in freier Form als auch in Form von Estern vorliegen können. Zu den Carotinoiden gehören beispielsweise β-Carotin, Canthaxanthin, Citranaxanthin, β-Apo-8′-Carotinsäureethylester, Astaxanthin, Xanthophylle, wie Lutein und Zeaxanthin, sowie Gemische dieser Carotinoide.

Die erfindungsgemäßen Gemische können aber auch andere, nicht oxidationsempfindliche Bestandteile enthalten.

Nachfolgend ist die Erfindung anhand von Ausführungs- und Anwendungsbeispielen näher erläutert.

Ausführungsbeispiel 1

220 Teile Gelatine, 36 Teile Zucker und 96 Teile Dextrin gab man in 600 Teile Wasser und löste sie unter Erhitzen auf 70°C. Andererseits machte man 220 Teile Vitamin A-Acetat durch Schmelzen homogen, dem 3,8 Teile Vitamin $D_3$ und 7,2 Teile BHT zugesetzt waren. Der so erhaltenen Mischung führte man 44 Teile Sojaöl und 72 Teile Ethoxyquin unter Rühren zu und erhielt eine homogene Mischung. Anschließend gab man zu der zuvor genannten wäßrigen Lösung 20 Teile 50 %ige Phytinsäure. Danach gab man die zuvor genannte Vitamin-Mischung zu und erhielt durch Dispergieren und Mischen eine Vitamin-Dispersion. Diese Dispersion leitete man in einen Sprühturm und versprühte sie. Dann trocknete man sie mit einem Wirbelbett-Trockner und erhielt ein stabiles Vitamin A/$D_3$-Pulver.

Ausführungsbeispiel 2

In 100 Teile Wasser wurden 53 Teile Gelatine und 113 Teile Glucosesirup gegeben und nach 30-minütigem Quellen der Gelatine durch Erhitzen auf 70°C in Lösung gebracht. Anschließend wurden nacheinander unterschiedliche Mengen eines Triglycerids und eines Komplexbildners sowie 50,1 Teile Vitamin A-Acetat zugegeben, das durch Zugabe von 4,5 mg BHT/Mio I.E. und 100 mg Ethoxyquin/Mio I.E. stabilisiert war. Das Gemisch wurde nach Zugabe von weiteren 70 Teilen Wasser durch kräftiges Rühren emulgiert. Die erhaltene Emulsion wurde schließlich bei 70°C mit Hilfe einer handelsüblichen Sprühpistole in eine mit hydrophober Kieselsäure beladene Stickstoffatmosphäre versprüht. Das erhaltene Produkt wurde anschließend auf einem Wirbelbett-Trockner im Stickstoffstrom bei Raumtemperatur getrocknet.

Gemäß dem angegebenen Ausführungsbeispiel 2 wurden folgende Menge Triglycerid und Komplexbildner in die Rezeptur eingesetzt (die angegebenen Gewichtsteile beziehen sich jeweils auf 100 Teile Trockenpulver; bei der Einstellung der Rezeptur wurde entsprechend der Zugabe von Zusätzen weniger Glucosesirup eingesetzt):

Ausführungsbeispiel 2-1

Keine Zugabe von Komplexbildner und Triglycerid.

Ausführungsbeispiel 2-2

Zugabe von 5 % Sojaöl, keine Zugabe eines Komplexbildners.

Ausführungsbeispiel 2-3

Zugabe von 5 % Erdnußöl, keine Zugabe eines Komplexbildners.

Ausführungsbeispiel 2-4

Zugabe von 5 % Maisöl, keine Zugabe eines Komplexbildners.

Ausführungsbeispiel 2-5

Zugabe von 1 % Phytinsäure, keine Zugabe von Triglycerid.

Ausführungsbeispiel 2-6

Zugabe von 1 % Phytinsäure, Zugabe von 5 % Sojaöl.

Ausführungsbeispiel 2-7

Zugabe von 1,5 % Phytinsäure, Zugabe von 5 % Sojaöl.

Ausführungsbeispiel 2-8

Zugabe von 1 % Phytinsäure, Zugabe von 2 % Sojaöl.

Ausführungsbeispiel 2-9

Zugabe von 1 % Phytinsäure, Zugabe von 10 % Sojaöl.

Ausführungsbeispiel 2-10

Zugabe von 1 % Phytinsäure, Zugabe von 5 % Erdnußöl.

Ausführungsbeispiel 2-11

Zugabe von 1 % Phytinsäure, Zugabe von 5 % Maisöl.

Ausführungsbeispiel 2-12

Zugabe von 1 % Natriumsalz der Phytinsäure, Zugabe von 5 % Sojaöl.

Ausführungsbeispiel 2-13

Zugabe von 1 % Calciumsalz der Phytinsäure, Zugabe von 5 % Sojaöl.

Ausführungsbeispiel 2-14

Zugabe von 1 % Zitronensäure, Zugabe von 5 % Sojaöl.

Ausführungsbeispiel 2-15

Zugabe von 1 % Natriumcitrat, Zugabe von 5 % Sojaöl.

Ausführungsbeispiel 2-16

Zugabe von 1 % Weinsäure, Zugabe von 5 % Sojaöl.

Ausführungsbeispiel 2-17

Zugabe von 1 % Natriumtartrat, Zugabe von 5 % Sojaöl.

Ausführungsbeispiel 2-18

Zugabe von 1 % Salicylsäure, Zugabe von 5 % Sojaöl.

Ausführungsbeispiel 2-19

Zugabe von 1 % Gluconsäure, Zugabe von 5 % Sojaöl.

Ausführungsbeispiel 2-20

Zugabe von 1 % Phosphorsäure, Zugabe von 5 % Sojaöl.

Ausführungsbeispiel 2-21

Zugabe von 1 % Natriumpyrophosphat, Zugabe von 5 % Sojaöl.

Ausführungsbeispiel 2-22

Zugabe von 1 % Natriumhexametaphosphat, Zugabe von 5 % Sojaöl.

Ausführungsbeispiel 2-23

Zugabe von 1 % Natriumtripolyphosphat, Zugabe von 5 % Sojaöl.

Ausführungsbeispiel 2-24

Zugabe von 1 % EDTA-Na(Titriplex® III), Zugabe von 5 % Sojaöl.

Ausführungsbeispiel 2-25

Zugabe von 1 % NTA-Na(Titriplex I), Zugabe von 5 % Sojaöl.

Ausführungsbeispiel 2-26

Zugabe von 1 % Cystein, Zugabe von 5 % Sojaöl.

Ausführungsbeispiel 2-27

Zugabe von 0,5 % Phytinsäure, Zugabe von 5 % Sojaöl.

Ausführungsbeispiel 2-28

Zugabe von 1 % Natriumphytat, Zugabe von 5 % Myglyol® 812 (synthetisches Fettsäuretriglycerid).

Ausführungsbeispiel 2-29

Zugabe von 1 % Natriumcitrat, keine Zugabe eines Triglycerids.

Ausführungsbeispiel 2-30

Zugabe von 1 % Phosphorsäure, keine Zugabe eines Triglycerids.

Ausführungsbeispiel 2-31

Zugabe von 1 % Natriumhexametaphosphat, Zugabe von 5 % Erdnußöl.

Ausführungsbeispiel 2-32

Zugabe von 1 % Natriumhexametaphosphat, keine Zugabe eines Triglycerids.

Ausführungsbeispiel 2-33

Zugabe von 1 % Triplex III, keine Zugabe eines Triglycerids.

Ausführungsbeispiel 2-34

Zugabe von 1 % Cystein, keine Zugabe eines Triglycerids.

Ausführungsbeispiel 3

52,3 Teile Gelatine wurden in 150 Teilen Wasser nach Zugabe von 126,8 Teilen Glucosesirup bei Raumtemperatur zum Quellen gebracht, danach wurde das Gemisch auf 65°C erhitzt. Nach Zugabe von 50 Teilen Tocopherol wurde 20 Min bei dieser Temperatur durch kräftiges Rühren emulgiert. Die Emulsion wurde anschließend über eine Einstoffdüse in eine mit hydrophober Kieselsäure beladene Stickstoffatmosphäre versprüht. Die gebildeten Produktteilchen wurden im Stickstoffstrom auf einer Glasfilternutsche getrocknet, bis der Restfeuchte-Gehalt einen Wert von 3 % erreicht hatte. Gemäß dem angegebenen Ausführungsbeispiel 3 wurden die folgenden Mengen eines Fettsäure-Triglycerids und eines Komplexbildners in die Rezeptur eingesetzt, wobei sich die angegebenen Gewichtsteile auf jeweils 100 Teile des erhaltenen Trockenpulvers bezie-

6

hen:

Ausführungsbeispiel 3-1

Keine Zugabe eines Komplexbildners oder eines Fettsäuretriglycerids bzw. Pflanzenöls.

Ausführungsbeispiel 3-2

Keine Zugabe eines Komplexbildners, Zugabe von 5 % Sojaöl.

Ausführungsbeispiel 3-3

Zugabe von 1 % Natriumhexametaphosphat, keine Zugabe eines Pflanzenöls.

Ausführungsbeispiel 3-4

Zugabe von 1 % Natriumhexametaphosphat, Zugabe von 5 % Sojaöl.

Ausführungsbeispiel 4

Gemäß Ausführungsbeispiel 3 wurden in die mit 122 Teilen Glucosesirup in 180 Teilen Wasser gequollene Gelatine (52,3 Teile) hergestellte Mischung nach dem Erwärmen auf 60°C, 52,4 Teile Vitamin A-Acetat (2,1 Mio I.E./g), stabilisiert mit 100 mg Ethoxyquin/Mio I.E. und 0,55 Teile Vitamin $D_3$ (40 Mio I.E./g) eingetragen und 20 Min emulgiert. Die erhaltene Emulsion wurde wie beschrieben versprüht und das erhaltene Produkt anschließend im Stickstoffstrom getrocknet. Gemäß dem angegebenen Ausführungsbeispiel 4 wurden zusätzlich zu den angegebenen Stoffen die folgenden Mengen eines Fettsäuretriglycerids und eines Komplexbildners in die Rezeptur eingesetzt, wobei sich die angegebenen Gewichtsteile auf jeweils 100 Teile des erhaltenen Trockenpulvers beziehen:

Ausführungsbeispiel 4-1

Keine Zugabe eines Komplexbildners oder eines Pflanzenöls.

Ausführungsbeispiel 4-2

Zugabe von 1 % Phytinsäure und 5 % Sojaöl.

Ausführungsbeispiel 5

Ausführungsbeispiel 5-1

Eine Citranaxanthindispersion bestehend aus

4,9 % Citranaxanthin (mikronisiert)

0,3 % Ascorbylpalmitat

1,6 % Ethoxyquin

14,6 % Gelatine 100 Bloom

13,1 % Saccharose

65,5 % Wasser wurde mit einer Temperatur von 50°C mit einer Einstoffdüse bei 6,0 bis 6,5 bar in einem Sprühturm in eine Wolke hydrophober Kieselsäure gesprüht. Das feuchte Produkt wurde auf einem Wirbeltrockner bei Raumtemperatur auf eine Restfeuchte von ca. 4 % getrocknet. Der Wirkstoffgehalt des Pulvers betrug 13,7 %.

Ausführungsbeispiel 5-2

Eine Citranaxanthindispersion bestehend aus

4,3 % Citranaxanthin (mikronisiert

0,3 % Ascorbylpalmitat

1,4 % Ethoxyquin

12,7 % Gelatine 100 Bloom

9,7 % Saccharose

1,6 % Sojaöl

70 % Wasser

wurde wie 5-1 behandelt. Der Wirkstoffgehalt des Pulvers lag bei 13,2 %.

Ausführungsbeispiel 5-3

Eine Citranaxanthindispersion bestehend aus

4,5 % Citranaxanthin (mikronisiert)

0,3 % Ascorbylpalmitat

1,5 % Ethoxyquin

13,2 % Gelatine 100 Bloom

9,7 % Saccharose

1,7 % Sojaöl

0,4 % Phytinsäure (als Na-Salz)

68,7 % Wasser

wurde wie 5-1 behandelt. Der Wirkstoffgehalt des Pulvers lag bei 13,2 %.

Ausführungsbeispiel 5-4

Eine Citranaxanthindispersion bestehend aus

4,8 % Citranaxanthin (mikronisiert)

0,3 % Ascorbylpalmitat

1,5 % Ethoxyquin

13,5 % Gelatine 100 Bloom

9,7 % Saccharose

1,8 % Sojaöl

0,4 % Ethylendiamintetraessigsäure (als Na-Salz)

wurde wie bei 5-1 behandelt. Der Wirkstoffgehalt des Pulvers lag bei 13,0 %.

Ausführungsbeispiel 6

Ausführungsbeispiel 6-1

200 Teile einer Dispersion, bestehend aus 106,8 Teilen Wasser, 1,1 Teilen Konservierungsstoffgemisches, 3,8 Teilen Ethoxyquin, 47,3 Teilen Saccharose, 29 Teilen Gelatine (Typ B 200) und 13,3 Teilen mikronisiertem Canthaxanthin wurden bei 40°C in 400 Teilen Paraffinöl unter kräftigem Rühren dispergiert. Nach dem Abkühlen auf 18°C wurde durch Zugabe von Maisstärke die Oberfläche der gebildeten Teilchen gepudert. Das Produkt, das jetzt in Form kleiner Kügelchen mit einem Durchmesser von 50 bis 500 μ vorlag, wurde mit 2000 Teilen kaltem Petrolether gewaschen und schließlich auf einem Wirbeltrockner bei Raumtemperatur auf einen Restfeuchtegehalt von 4 % getrocknet. Der Wirkstoffgehalt des Pulvers lag bei 10,7 %.

Ausführungsbeispiel 6-2

Gemäß Ausführungsbeispiel 6-1 wurden in die Emulsion zusätzlich 4,0 Teile Sojaöl und 0,8 Teile Phytinsäure gegeben. Nach der Aufarbeitung der Dispersion gemäß dem angeführten Ausführungsbeispiel 6-1 wurde ein Pulver erhalten mit einem Canthaxanthingehalt von 10,1 %, mit 1 % Phytinsäure und 5 % Sojaöl.

Anwendungsbeispiel 1

Prüfung von Vitamin A/D$_3$-haltigen Trockenpulvern

1,0 g Pulver des Ausführungsbeispiel 1 füllte man in eine Glasflasche ohne Zusatz von Prämix, lagerte diese bei einer konstanten Temperatur von 40°C und 70 % relative Luftfeuchte und maß in zeitlichen Abständen den verbliebenen Anteil des Vitamin A. Das Ergebnis ist in der folgenden Tabelle aufgezeigt.

10 g Pulver des Ausführungsbeispiels 1 mischte man mit 40 g Prämix-Substrat, bestehend aus 60 % Weizenmehl (wheat middlings), 30 % 50 %igem Cholinchlorid und 10 % einer Spurenelementmischung. 3,2 g des so erhaltenen Prämix füllte man in eine Glasflasche, deckte sie mit einer Aluminiumfolie statt eines Deckels ab und machte 5 Löcher in die Aluminiumfolie zur Belüftung. Diese Flasche stellte man in einen Behälter mit der konstanten Temperatur von 40°C und der konstanten relativen Luftfeuchte von 70 % und maß den verbliebenen Anteil (Retention %) des Vitamin A nach bestimmten Zeiten. Die oben genannten Spurenelemente waren ein Gemisch aus 37,44 % $CuSO_4$ x 5 $H_2O$, 46,78 % $FeSO_4$ x 7 $H_2O$, 11,78 % ZnO; 3,61 % MnO und 0,39 % $CoCO_3$.

Die Prüfungsergebnisse sind in der folgenden Tabelle I aufgezeigt:

Tabelle I

| Versuch | Tage | Vitamin A-Retention (%) | |
|---|---|---|---|
| | | nach 1 Monat (%) | nach 2 Monaten (%) |
| Pulver des Ausführungs- beispiels 1 alleine | 100 | 90 | 86 |
| Prämix vermischt mit dem Pulver des Ausführungs- beispiels 1 | 100 | 63 | 52 |
| Pulver ähnlich dem des Ausführungsbeispiels 1, aber ohne Sojaöl und Phytinsaüre | 100 | 59 | 40 |

Anwendungsbeispiel 2

Prüfung von Vitamin A-haltigen Trockenpulvern

1 Gew.% der einzelnen Pulver des Ausführungsbeispiels 2 mischte man mit 99 % des in Anwendungsbeispiel 1 genannten Prämix. 4 g Proben dieser Mischungen wurden am Anfang und nach 4 Wochen Lagerung in einem Klimaschrank bei 40°C und 70 % relativer Feuchte auf ihren Vitamin A-Gehalt geprüft. Der nach 4 Wochen verbliebene Anteil ist in % vom Ausgangswert der Lagerung in folgenden Tabellen II und III angegeben.

Tabelle II

Vitamin A (zu Anwendungsbeispiel 2)

| Ausführungs-beispiel Nr. | Komplexbildner | Öl | Retention (%) nach 4 Wochen |
|---|---|---|---|
| 2- 1 | – | – | 17 |
| 2- 2 | – | 5 % Sojaöl | 32 |
| 2- 3 | – | 5 % Erdnußöl | 31 |
| 2- 4 | – | 5 % Maisöl | 46 |
| 2- 5 | 1 % Phytinsäure | – | 24 |
| 2- 6 | 1 % Phytinsäure | 5 % Sojaöl | 47 |
| 2- 7 | 1,5 % Phytinsäure | 5 % Sojaöl | 53 |
| 2- 8 | 1 % Phytinsäure | 2 % Sojaöl | 55 |
| 2- 9 | 1 % Phytinsäure | 10 % Sojaöl | 59 |
| 2-10 | 1 % Phytinsäure | 5 % Erdnußöl | 53 |
| 2-11 | 1 % Phytinsäure | 5 % Maisöl | 54 |
| 2-12 | 1 % Natriumphytat | 5 % Sojaöl | 58 |
| 2-13 | 1 % Calciumphytat | 5 % Sojaöl | 59 |
| 2-14 | 1 % Zitronensäure | 5 % Sojaöl | 46 |
| 2-15 | 1 % Natriumcitrat | 5 % Sojaöl | 53 |
| 2-16 | 1 % Weinsäure | 5 % Sojaöl | 49 |
| 2-17 | 1 % Natriumtartrat | 5 % Sojaöl | 55 |
| 2-18 | 1 % Salicylsäure | 5 % Sojaöl | 53 |
| 2-19 | 1 % Gluconsäure | 5 % Sojaöl | 50 |
| 2-20 | 1 % Phosphorsäure | 5 % Sojaöl | 63 |
| 2-21 | 1 % Natriumpyrophosphat | 5 % Sojaöl | 57 |
| 2-22 | 1 % Natriumhexametaphosphat | 5 % Sojaöl | 53 |
| 2-23 | 1 % Natriumtripolyphosphat | 5 % Sojaöl | 57 |
| 2-24 | 1 % EDTA Natrium (TitriplexIII) | 5 % Sojaöl | 59 |
| 2-25 | 1 % NTA Natrium (Titriplex I) | 5 % Sojaöl | 51 |
| 2-26 | 1 % Cystein | 5 % Sojaöl | 59 |
| 2-27 | 0,5 % Phytinsäure | 5 % Sojaöl | 29 |

Tabelle III

Vitamin A (zu Anwendungsbeispiel 2)

| Ausführungs-beispiel Nr. | Komplexbildner | Öl | Retention (%) nach 4 Wochen |
|---|---|---|---|
| 2-28 | 1 % Natriumphytat | 5 % synth. Triglycerid*) | 54 |
| 2-29 | 1 % Natriumcitrat | – | 13 |
| 2-30 | 1 % Phosphorsäure | – | 55 |
| 2-31 | 1 % Natriumhexametaphosphat | 5 % Erdnußöl | 44 |
| 2-32 | 1 % Natriumhexametaphosphat | – | 28 |
| 2-33 | 1 % EDTA Natrium (TitriplexIII) | – | 20 |
| 2-34 | 1 % Cystein | – | 25 |

*) Myglyol 812 (Neutralöl) = Capryl-Caprinsäuretriglycerid

Anwendungsbeispiel 3

Prüfung von Tocopherol-haltigen Trockenpulvern

1 Gew.% der einzelnen Pulver des Ausführungsbeispiels 3 mischte man mit 99 % des in Anwendungsbeispiel 1 genannten Prämix. 6 g Proben dieser Mischungen wurden am Anfang und nach 4 Wochen Lagerung in einem Klimaschrank bei 40°C und 70 % r.F. auf ihren Tocopherolgehalt geprüft. Der nach 4 Wochen verbliebene Anteil ist in Prozent vom Ausgangswert der Lagerung in folgender Tabelle angegeben.

| Ausführungs-beispiel Nr. | Komplexbildner | Öl | Retention (%) nach 4 Wochen |
|---|---|---|---|
| 3-1 | – | – | 18 |
| 3-2 | – | 5 % Sojaöl | 23 |
| 3-3 | 1 % Natriumhexametaphosphat | – | 41 |
| 3-4 | 1 % Natriumhexametaphosphat | 5 % Sojaöl | 49 |

Anwendungsbeispiel 4

Prüfung von Vitamin $D_3$-haltigen Trockenpulvern

Vitamin A/$D_3$ Trockenpulver des Ausführungsbeispiels 4 wurde im gleichen Prämix wie in Anwendungsbeispiel 5 geprüft. Es wurden 2 Mio I.E. Vitamin $D_3$/kg Prämix eingemischt. Je 10 g Proben der fertigen Mischungen wurden am Anfang nach 4 und 8 Wochen Lagerung in einem Klimaschrank bei 40°C und 70 % rel. Feuchte auf ihren Vitamin $D_3$-Gehalt geprüft. Der nach 8 Wochen verbliebene Anteil ist in Prozent vom Ausgangswert der Lagerung in nachstehender Tabelle angegeben.

11

| Ausführungs-<br>beispiel Nr. | Komplexbildner | Öl | Retention (%)<br>nach 4 Wochen |
|---|---|---|---|
| 4-1 | - | - | 40 |
| 4-2 | 1 % Phytinsäure | 5 % Sojaöl | 81 |

Anwendungsbeispiel 5

Prüfung von Carotinoid-haltigen Trockenpulvern

Carotinoid-haltige Trockenpulver der Ausführungsbeispiele 5 und 6 wurden in einem Prämix folgender Zusammensetzung auf ihre Stabilität geprüft: 76 % Weizengrießkleie, 13,3 % Cholinchlorid flüssig (mit 75 % Cholinchlorid und 25 % Wasser) und 10 % der Spurenelementmischung wie in Anwendungsbeispiel 1. Der Prämix lagerte 3 Tage, bevor 1 Gew.% der Carotinoid-Trockenpulver eingemischt wurde. 1 g Proben der fertigen Mischungen wurden am Anfang und nach 4 Wochen Lagerung in einem Klimaschrank bei 40°C und 70 % rel. Feuchte auf ihren Carotinoid-Gehalt geprüft. Der nach 4 Wochen verbliebene Anteil ist in Prozent vom Ausgangswert der Lagerung in nachstehender Tabelle angegeben.

| Ausführungs-<br>beispiel Nr. | Wirkstoff | Komplexbildner | Öl | Retention (%)<br>nach 4 Wochen |
|---|---|---|---|---|
| 5-1 | Citranaxanthin | - | - | 22 |
| 5-2 | Citranaxanthin | - | 5 % Sojaöl | 48 |
| 5-3 | Citranaxanthin | 1 % Natriumphytat | 5 % Sojaöl | 68 |
| 5-4 | Citranaxanthin | 1 % EDTA-Natrium | 5 % Sojaöl | 63 |
| 6-1 | Canthaxanthin | - | - | 33 |
| 6-2 | Canthaxanthin | 1 % Phytinsäure | 5 % Sojaöl | 79 |

**Patentansprüche**

1. Stabiles Gemisch mit einem Gehalt oxidationsempfindlicher Verbindungen, enthaltend die oxidationsempfindlichen Verbindungen, Triglyceride, Komplexbildner sowie Umhüllungsstoffe.

2. Gemisch nach Anspruch 1, zusätzlich enthaltend Antioxidantien.

3. Gemisch nach einem der Ansprüche 1 oder 2, worin es sich bei dem Triglycerid um ein Pflanzenöl handelt.

4. Gemisch nach einem der Ansprüche 1 bis 3, worin es sich bei dem Komplexbildner um Phytinsäure, Phosphorsäure, Meta-, Pyro- oder Polyphosphorsäure oder um deren Salze handelt.

5. Gemisch nach einem der Ansprüche 1 bis 4, worin es sich bei dem Umhüllungsstoff um einen Filmbildner, insbesondere Proteine, Zucker oder Polysaccharide, insbesondere Gummi arabicum, Alginate, Stärkederivate, handelt.

6. Gemisch nach einem der Ansprüche 1 bis 5, worin es sich bei der oxidationsempfindlichen Verbindung um fettlösliche Vitamine, insbesondere Vitamin A und dessen Ester, Vitamin A-säure, Vitamin E, Vitamin D, um Carotinoide, oder Riech- und Aromastoffe handelt.

7. Gemisch nach einem der Ansprüche 1 bis 6, enthaltend Vitamin A oder einen seiner Ester, Phytinsäure, Pflanzenöl und Gelatine.

8. Gemisch nach einem der Ansprüche 1 bis 7, in Form eines Granulats oder Pulvers mit einem Korngrößenbereich zwischen 100 und 600 μm.

9. Verfahren zur Herstellung des Gemischs nach einem der Ansprüche 1 bis 8, dadurch gekennzeichnet, daß man eine Dispersion aus oxidationsempfindlicher Verbindung, Triglycerid, Komplexbildner und Umhüllungsstoff herstellt und diese Dispersion in einen Feststoff überführt.

10. Verfahren nach Anspruch 9, dadurch gekennzeichnet, daß man eine wäßrige Dispersion herstellt, trocknet und granuliert oder pulverisiert.

11. Verwendung einer Kombination aus Triglycerid, Komplexbildner und Umhüllungsstoff zur Stabilisierung oxidationsempfindlicher Verbindungen.

## Claims

1. A stable mixture containing an oxidation-sensitive compound, a triglyceride, a complexing agent and a coating substance.

2. A mixture as claimed in claim 1, which additionally contains an antioxidant.

3. A mixture as claimed in either of claims 1 and 2, wherein the triglyceride is a vegetable oil.

4. A mixture as claimed in any of claims 1 to 3, wherein the complexing agent is phytic acid, phosphoric acid, meta-, pyro- or polyphosphoric acid or a salt thereof.

5. A mixture as claimed in any of claims 1 to 4, wherein the coating substance is a film former, in particular a protein, sugar or polysaccharide, in particular gum arabic, an alginate or a starch derivative.

6. A mixture as claimed in any of claims 1 to 5, wherein the oxidation-sensitive compound is a fat-soluble vitamin, in particular vitamin A or an ester thereof vitamin A acid, vitamin E or vitamin D, a carotenoid or a scent or flavoring substance.

7. A mixture as claimed in any of claims 1 to 6, containing vitamin A or an ester thereof, phytic acid, a vegetable oil and gelatin.

8. A mixture as claimed in any of claims 1 to 7, in granular or pulverulent form having a particle size range of from 100 to 600 μm.

9. A process for preparing a mixture as claimed in any of claims 1 to 8, which comprises preparing a dispersion from an oxidation-sensitive compound, a triglyceride, a complexing agent and a coating substance and converting this dispersion into a solid substance.

10. A process as claimed in claim 9, wherein an aqueous dispersion is prepared, dried and granulated or pulverized.

11. Use of a combination of a triglyceride, a complexing agent and a coating substance for stabilizing an oxidation-sensitive compound.

## Revendications

1. Mélange stable ayant une teneur en composés sensibles à l'oxydation, contenant les composés sensibles à l'oxydation, des triglycérides, des complexants, ainsi que des matières d'enrobage.

2. Mélange selon la revendication 1, contenant en outre des antioxydants.

3. Mélange selon la revendication 1 ou 2, dans lequel il s'agit, en ce qui concerne le triglycéride, d'une huile végétale.

4. Mélange selon l'une quelconque des revendications 1 à 3, dans lequel il s'agit, en ce qui concerne le complexant, d'acide phytinique, d'acide phosphorique, d'acide méta-, pyro- ou polyphosphorique ou de leurs sels.

5. Mélange selon l'une quelconque des revendications 1 à 4, dans lequel il s'agit, en ce qui concerne la matière d'enrobage, d'un filmogène, en particulier de protéines, de sucres ou de polysaccharides, en particulier de gomme arabique, d'alginates, de dérivés d'amidon.

6. Mélange selon l'une quelconque des revendications 1 à 5, dans lequel il s'agit, en ce qui concerne le composé sensible à l'oxydation, de vitamines liposolubles, en particulier de vitamine A et de ses esters, d'acide de vitamine A, de vitamine E, de vitamine D, de caroténoïdes, ou de substances odorantes et aromatiques.

7. Mélange selon l'une quelconque des revendications 1 à 6, contenant de la vitamine A ou l'un de ses esters, de l'acide phytinique, de l'huile végétale et de la gélatine.

8. Mélange selon l'une quelconque des revendications 1 à 7, sous forme d'un granulé ou d'une poudre ayant une grosseur de grains dans la gamme comprise entre 100 et 600 μm.

9. Procédé de préparation du mélange selon l'une quelconque des revendications 1 à 8, caractérisé en ce qu'on prépare une dispersion du composé sensible à l'oxydation, du triglycéride, du complexant et de la matière d'enrobage, et on transforme cette dispersion en une matière solide.

10. Procédé selon la revendication 9, caractérisé en ce qu'on prépare une dispersion aqueuse, on la sèche et on la granule ou on la pulvérise.

11. Utilisation d'une combinaison de triglycéride, de complexant et de matière d'enrobage pour la stabilisation de composés sensibles à l'oxydation.